# EUROPEAN PATENT SPECIFICATION

(11) **EP 1 185 621 B1**
(45) Date of publication and mention of the grant of the patent: **24.02.2010**
(21) Application number: 00940540.8
(22) Date of filing: 23.06.2000
(51) Int. Cl.: C12N 5/00, A61K 35/12, A61K 8/00

(54) **SURFACE FOR CELL CULTURE**
OBERFLÄCHE FÜR ZELLKULTUR
SURFACE DE DECOLLEMENT

(30) Priority: 23.06.1999 GB 9914616
(43) Date of publication of application: 13.03.2002
(73) Proprietor: Altrika Limited, Edinburgh EH3 8AE (GB)
(72) Inventor: SHORT, Robert, University of Sheffield, Sheffield S1 3JD (GB); HADDOW, David, University of Sheffield, Sheffield S1 3JD (GB); MACNEIL, Sheila, Northern General Hospital, Sheffield S5 7AU (GB)
(74) Representative: Hutter, Anton
(86) International application number: PCT/GB2000/002297
(87) International publication number: WO 2000/078928

(56) References cited:
- EP-A- 0 101 285
- WO-A-87/05038
- R.M. FRANCE ET AL.: "Attachment of human keratinocytes to plasma co-polymers of acrylic acid/octa-1,7-diene and allyl amine/octa-1,7,-diene." JOURNAL OF MATERIALS CHEMISTRY, vol. 8, no. 1, 1998, pages 37-42, XP002158573 cited in the application
- R. DAW ET AL.: "Plasma copolymer surfaces of acrylic acid/1,7 octadiene: Surface characterisation and the attachment of ROS 17/2.8 osteoblast-like cells." BIOMATERIALS, vol. 19, 1998, pages 1717-1725, XP002158574 GUILDFORD, GB
- D.B. HADDOW ET AL.: "Comparison of proliferation and growth of human keratinocytes on plasma copolymers of acrylic acid/1,7-octadiene and self-assembled monolayers." JOURNAL OF BIOMEDICAL MATERIALS RESEARCH, vol. 47, no. 1, October 1999 (1999-10), pages 379-387, XP000978964 NEW YORK, US cited in the application

## Description

The invention relates to a surface to which cells, preferably mammalian cells, attach and proliferate and which enables said attached cells to detach from the surface which can be used in various therapeutic and cosmetic tissue engineering/surgical procedures.

Tissue engineering is an emerging science which has implications with respect to many areas of clinical and cosmetic surgery. More particularly, tissue engineering relates to the replacement and/or restoration and/or repair of damaged and/or diseased tissues to return the tissue and/or organ to a functional state. For example, and not by way of limitation, tissue engineering is useful in the provision of skin grafts to repair wounds occuring as a consequence of: contusions, or bums, or failure of tissue to heal due to venous or diabetic ulcers.

Unfortunately, the culturing of cells/tissues *in vitro* represents only part of the problem faced by tissue engineers. In many examples the growth of cells in culture is not the major obstacle. It is the transfer of the cells/tissue, via a suitable vehicle ( for example and not by way of limitation culture wear, prostheses, implants, 3-dimensional matrix supports, extracellular matrix protein coated dressing, bandages, plasters), so that the cells/tissue are incorporated into the patient to be treated which represents a further, more taxing problem. Vehicles suitable for the transfer of replacement tissue have to satisfy certain requirements if they are to be useful in tissue engineering. For example, transfer vehicles optionally have the following characteristics;
i) they provide a surface to which cells may become securely attached;
ii) they allow attached cells to grow and divide unhindered by the attachment surface;
iii) where appropriate, they provide an attachment surface which does not influence the differentiated (or undifferentiated) state of the attached cells;
iv) they maintain cells in a sterile and immunologically silent status;
v) they are minimally toxic to the patient;
vi) they do not transmit bacterial or viral disease; and
vii) they provide a surface from which attached cells may easily detach and subsequently invade the tissue site requiring replacement, restoration or repair.

A number of surfaces have been identified which provide substrates on which cells may attach, grow and proliferate in culture and an excellent example of a cell type expressing the aforementioned characteristics is a keratinocyte.

The favoured substratum for supporting the attachment and proliferation and growth of cells is collagen I, but others have been investigated (1). For example, keratinocytes seeded or deposited onto collagen-glycosaminoglycan (C-GAG) substrates and grafted to burns to form a cultured skin substitute (CSS), developed into permanent skin tissue after 14-28 days (2). Keratinocytes are also able to grow *in vitro* on synthetic hydrophilic polymer supports (3). Keratinocytes have been grafted onto poly(hydroxyethyl methacrylate) supports and these have shown improved wound bed healing, with no difference in the cytokeratin pattern of the unreconstructed epidermis and normal human skin(4). Previous work has shown how carboxylic acid-containing plasma co-polymers encouraged the attachment and proliferation of keratinocytes over a period of 7 days (5).

The influence of extracellular matrix proteins on human keratinocyte attachment, proliferation and transfer to a dermal wound bed model has also been studied (6). Matrigel, collagen I and IV were found to enhance initial attachment; RGD, vitronectin, fibronectin and irradiated 3T3 fibroblasts did not. Proliferation of cells was also found to be positively influenced (although to a lesser extent than initial attachment) on matrigel, collagen I and IV and irradiated 3T3 fibroblasts. Keratinocytes proliferating on the latter substrates maintained the ability to transfer to a simple *in vitro* wound bed model.

The culture of cells on defined-surfaces has rarely addressed the challenges of differentiated cells. Fibroblasts, for example, commonly used in studies on differentiated cell types, maintain considerable pleiotropy in culture and are relatively easy to culture. Keratinocytes in contrast will normally undergo programmed differentiation *in vivo* moving upwards from the basal epidermal layer (where they are in contact with the basement membrane attached to the underlying dermis) to the upper epidermal layers. In the latter layers they lose their nuclei and undergo terminal differentiation. Once past a certain point of differentiation they are not capable of migration or proliferation and largely serve a barrier function for the skin.

Keratinocytes in culture undergo terminal differentiation in most culture conditions and will react to adverse conditions by premature differentiation. The challenge therefore is to establish a surface which, ideally, does not itself promote differentiation and which can, ideally in conjunction with appropriate culture media, maintain cells, such as keratinocytes, in a proliferative phenotype in which they are capable of attaching and then subsequently transferring to an *in vitro* wound bed model.

When considering tissue engineering and wound repair, several different approaches are available. Products and potential therapies currently being investigated generally fall into three categories: epidermal replacements, dermal replacements and skin substitutes.

Epidermal replacements consist of keratinocytes cultured as a sheet alone, or with a vehicle, and usually involve culturing autologous (patient's own) epithelial cells grown to confluence *in vitro.* Although non-autologous versions are available as "off the shelf" solutions, there is no evidence that non-autologous cells will be compatible, although they can act as a biological bandage. For these reasons, non-autologous, products (Epicel^{tm} and Acticel^{tm}) have received mixed clinical success. Another product under investigation, Laserskin, uses hyaluronic acid as a keratinocyte delivery system, but when used by those skilled in the art, the carrier is primed with a layer of irradiated 3T3 fibroblasts to promote keratinocyte proliferation. Others are also investigating film carriers for keratinocyte transfer prior to them forming an intact sheet.

Dermal replacements comprise a support structure, or implanted matrix, for infiltration, adherence, proliferation and neo-matrix production by fibroblasts (and in some cases endothelial cells). Integra^{tm} uses a dermal component of bovine dermal collagen I crosslinked with chrondroitin-6-sulphate on a silicone backing sheet. Also under consideration is a seeded variant with fibroblasts and epidermal cells. The synthetic matrix degrades after 3/4 weeks and promotes neo-dermis formation prior to split-thickness mesh grafting. Alloderm^{tm} is freeze-dried, human de-epidermised dermis containing donor fibroblasts ( from screened skin bank donors). Xenoderm^{tm} is similar, utilising a porcine dermis, which allows incorporation of the matrix into the wound bed, exhibits low immunogenicity and allows re-population with host cells. Others are also developing collagen based polymers as supports, or synthetic matrices, for the delivery of keratinocytes and fibroblasts which have been shown to support cell ingrowth when implanted unseeded into experimental wounds.

Currently considered the most promising product, Dermagraft^{tm} utilises a PGA/PLA matrix seeded with allogenic fibroblasts. Complete resorption of the implanted matrix, after 4 weeks is seen, and cells deposit collagen I-III-VI, elastin, fibronectin and decorin. An unseeded version didn't support a graft take. The advantage of this product is that it can retard wound contraction if seeded with keratinocytes, and the non collageneous matrix overcomes problems associated with immunogenicityBSE transfer.

Skin substitutes combine both the dermal and epidermal replacements. Appligraf^{tm} combines collagen I gel seeded with allogenic fibroblasts with a confluent sheet of allogenic keratinocytes. Although there are questions about the long term survival of allogenic keratinocytes and fibroblasts in dermal lesions, it is possible that viable allogenic cells may deliver biological mediators (e.g. growth factors) capable of accelerating the repair process.

It will be apparent to one skilled in the art that the above solutions to providing wound healing systems (other than those which use material derived from the patients tissue, ie autologous tissue) suffer from the potential to transfer infective agents from the donating source to the patient to be treated. Additionally, xenografts still require general acceptance by the general public as an alternative to the use of human tissue in tissue engineering.

Several issues concerning wound pre-treatment, choice of matrix support (for cell growth) and the use of allogenic cells remain to be fully resolved, but there is little doubt that tissue engineered approaches to wound repair will present significant therapeutic benefits compared with existing treatments. It will be apparent from the above description, that keratinocytes provide an excellent model system for the study of tissue for use in tissue engineering. However an overriding problem faced by tissue engineers is the provision of a substrate which can satisfy all the requirements of the ideal vehicle for the culture and transfer of cells/tissues to a patient.

Cell culture ware and biological implants or vehicles are typically manufactured from or coated with polymers which allow the attachment, growth and proliferation of cells. Often, if the substrate/vehicle is to be used as means to implant the cultured cells/tissues, then the implanted matrix used in conjunction therewith may be biodegradable (please see WO/9012603). Furthermore the treatment of substrates to encourage the attachment and proliferation of cells is well known in the art. For example WO89/02457 and WO90/0214 disclose the chemical modification of surfaces that facilitate the attachment of cells. WO89/02457 relates to the chemical modification of polytetrafluoroethylene (Teflon^{tm}) and other fluorocarbon polymers and their use in the culturing of endothelial cells. WO90/02145 describes the use of a co-polymer of neutralised perfluoro-3,6-dioxa-4-methyl-7-octene sulphonyl fluoride and the monomer tetrafluoroethylene for use in coating various types of substrate for use in cell/tissue culture.

US 4 919 659 describes the use of plasma polymerizable gases (eg acetone, methanol, ethylene oxide) to coat surfaces to which cells attach and grow. The coated surfaces show enhanced binding of fibronectin ( a cell adhesion polypeptide) and hence facilitate the attachment of cells to the treated surfaces. The surfaces thus treated are useful in providing articles for biological implants and cell culturewear. Typically, materials such as polyester, polytetrafluoroethylene or polyurethane are coated with a plasma polymerized gas and then contacted with fibronectin. The fibronectin adsorbed surfaces show enhanced attachment of mouse 3T3 cells when compared to control surfaces.

Plasmas are ionised gases, commonly excited by means of an electric field. They are highly reactive chemical environments comprising ions, electrons, neutrals (radicals, metastables, ground and excited state species) and electromagnetic radiation. At reduced pressure a regime may be achieved where the temperature of the electrons differs substantially from that of the ions and neutrals. Such plasmas are referred to as "cold" or "non-equilibrium" plasmas. In such an environment many volatile organic compounds (neat or with other gases, eg Ar) have been shown to polymerise (H. K. Yasuda, Plasma Polymerisation, Academic Press, London, 1985) coating both surfaces in contact with the plasma and those downstream of the discharge. The organic compound is often referred to as the "monomer". The deposit is often referred to as a "plasma polymer". The advantages of such a mode of polymerisation include: ultra-thin pinhole free film deposition; plasma polymers can be deposited onto a wide range of substrates; the process is solvent free and the plasma polymer is free of contamination.

We have exploited plasma polymer deposition to coat suitable substrates for use in cell/tissue culture (5,7,8). France et al. (J. Mater. Chem., 1998, 8(1), 37-42) discloses a study of the attachment of human keratinocytes to plasma copolymers of acrylic acid/octa-1,7-diene and allyl amine/octa-1,7-diene. Daw et al. (Biomaterials, 19, 1998, 1717-1725) discloses plasma copolymer surfaces of acrylic acid/1,7 octadiene and the attachment of osteoblast-like cells thereto.

Thin polymeric films can be obtained from the plasmas of volatile organic compounds ( at reduced pressure of 10⁻² mmbar and ideally, < 100° C). In plasma polymer deposition, there is generally extensive fragmentation of the starting compound or ionised gas and a wide range of the resultant fragments or functional groups are undesirably incorporated into the deposit. We have shown that by employing a low plasma input power (low plasma power/monomer flow rate ratio) it is possible to fabricate films with a high degree of functional group retention. An example of such a low power/rate ratio is 2W/2.0sccm. However, other relatively low ratios may be used and are known to those skilled in the art.

This has been demonstrated for acrylic acid (9). Co-poiymerisation of acrylic acid with a hydrocarbon (e.g. 1,7-octadiene) allows a degree of control over surface functional group concentrations in the resultant plasma copolymer (PCP) (7). PCPs can be deposited directly onto most surfaces, regardless of geometry, making them ideal for treating surfaces such as gauzes and fibres, as well as plasticware for cell culture. This would obviously make them useful for clinical applications where cells could be grown on PCP-coated 2-dimensional or 3-dimensional supports prior to application to wound beds or sites of tissue repair/restoration.

We have cultured human keratinocytes on surfaces which have been coated with plasma polymer/co-polymer. The use of a low power/monomer flow rate ratio produces a plasma polymer/co-polymer in which the acid functionality of the acid-containing monomer (in this example, acrylic acid) is largely preserved intact (retained) from the plasma-gas to the plasma polymer/co-polymer deposit. These deposits do contain other functional groups (e.g. hydroxyls arising from post plasma oxidation) but are described as "high acid functionality", reflecting the high degree of acid retention from the plasma gas into the plasma polymer film. "High acid functionality" does not refer to the amount (concentration/density) of acid functionality in the plasma polymer/co-polymer, which depends upon the co-polymerisation ratio of the acid-containing monomer/hydrocarbon.

Keratinocytes cultured on these surfaces not only attach, grow and proliferate in an undifferentiated state but also detach from the surface and transfer to a wound bed model. Surfaces that promote keratinocyte transfer in this manner show great promise in the field of wound healing. We ascribe these favourable characteristics to the high acid functionality of our treated surfaces and to the nature of the attachment surfaces in facilitating detachment of cells

Reference herein to high acid functionality is intended to include surfaces which have amounts of 5-20% surface acid functionality and more ideally in excess of 20% surface acid functionality. The percentages refer to the percent of carbon atoms in this type of environment. For example, 20 % acid functionality means that 20 of every one hundred carbons in the plasma polymer is in an acid-type environment.

According to a first aspect of the invention there is provided a therapeutic vehicle, wherein said vehicle has integral therewith, or applied thereto, a cell culture surface obtained by plasma polymerisation or plasma co-polymerisation, to which at least one cell is reversibly attached, wherein the surface contains an acid functionality of at least 5% and the cell is a cell type selected from any one of the following cell types: keratinocytes, endothelial cells and epithelial cells, for use as a medicament.

Reference herein to high acid functionality relates to surfaces which contain between 5-20% or greater than 20% surface acid functionality.

It will be apparent to one skilled in the art that an acid functionality at attachment surfaces results in enhanced attachment of cells (5,7,10-12). We have found that plasma polymerisation of cell culture surfaces at low plasma power/monomer flow rate ratio results in a retention of high acid functionality at surfaces coated with the polymer. Cells cultured on cell culture surfaces treated with 100% acrylic acid exhibit enhanced detachment from the treated surface thereby promoting keratinocyte infiltration of de-epidermised dermis (DED) The plasma polymer produced from 100% acrylic acid may not contain the optimal percentage of acid functionality for cell attachment. However, plasma co-polymerisation of acrylic acid with hydrocarbon, for example and not by way of limitation, 1,7-octadiene, allows a degree of control over the deposition process and the provision of a surface to which keratinocytes may attach, proliferate and detach therefrom. Typically, cell culture surfaces which have been treated with an excess of 50% acrylic acid in the monomer flow produce plasma polymer surfaces with between 5-21% acid functionality, depending on the concentration of acid used. For example, a surface treated with 100 % acrylic acid produces an acid surface functionality of approximately 21%.

In a preferred embodiment of the invention said surface provides a substrate onto which at least one cell can grow and proliferate. Preferably said surface facilitates growth and proliferation of said cell in an undifferentiated state. Alternatively, said surface facilitates the growth and proliferation of said cells in a differentiated state, depending on tissue type.

In yet a further preferred embodiment of the invention said surface does not elicit an immune reaction in the cells attached thereto so that these do not provoke an immune reaction when they are delivered to a patient.

In yet still a further preferred embodiment of the invention said surface has minimal patient toxicity and so does not elicit an unfavourable reaction when cells attached thereto are delivered to a patient.

In yet a further preferred embodiment of the invention said surface is suitable for use with cells of mammalian origin, and more preferably cells of human origin.

In a further preferred embodiment of the invention said surface is suitable for use with any one of the following cell types; keratinocytes; chondrocytes; osteoblasts; endothelial cells. Ideally said cell is a keratinocyte.

In yet a further preferred embodiment of the invention said surface acid functionality is provided by a carboxylic acid functionality

In yet still a further preferred embodiment of the invention said surface acid functionality is at least 5% and more usually between 5-20% surface acid functionality. More ideally still said surface acid functionality is greater than 20%. Ideally said acid functionality is provided by acrylic acid. Alternatively said acid functionality is provided by propionic acid.

In yet still a further preferred embodiment of the invention , typically said surface is provided by coating a substrate with a plasma co-polymer of an acidic monomer. For example and not by way of limitation, acrylic acid and a hydrocarbon, for example and not by way of limitation, 1,7-octadiene. Ideally said acrylic acid is provided at 50-100% and 1,7-octadiene is provided at 0-50% in the gas feed.

It will be apparent to one skilled in the art, that the cell culture surfaces of the invention are useful in clinical applications where cells could be grown on coated substrates prior to application to, for example and not by way of limitation, acute and/or chronic and/or minor and/or severe cutaneous wounds (including venous and diabetic ulcers); and/or cartilage repair; and/or bone repair; and/or muscle repair; and/or nerve repair; and/or connective tissue repair; and/or blood vessel repair; and/or bladder repair. The invention also provides any of the aforementioned cell culture surfaces by providing said surfaces as an integral part of a tissue engineering vehicle.

According to a second aspect of the invention there is provided a therapeutic vehicle, for use in wound healing, wherein said vehicle has integral therewith, or applied thereto, a cell culture surface obtained by plasma polymerisation or plasma co-polymetisation, to which at least one cell is reversibly attached, wherein the surface contains an acid functionality of at least 5% and the cell is a cell type selected from any one of the following cell types: keratinocytes, endothelial cells and epithelial cells.

The vehicle may be defined as in the dependent claims.

Vehicle may be defined as any structure by which cells cultured on a surface according to the invention may be used in tissue engineering. For example and not by way of limitation, a prosthesis, implant, matrix, stent, cell culture dishes, gauze, bandage, plaster, biodegradable matrix and polymeric film.

In a preferred embodiment of the invention there is provided a therapeutic vehicle comprising a surface according to the invention to which is attached selected cell(s) wherein said therapeutic vehicle is adapted to be applied and/or implanted into a patient requiring therapeutic tissue engineering.

In yet a further preferred embodiment of the invention there is provided a therapeutic vehicle comprising a matrix material ( for example, and not by way of limitation a matrix material which is synthetic or naturally occuring and either long-lasting or biodegradable) comprising a surface according to the invention to which is attached cells for use in surgical implantation procedures.

In yet still a further preferred embodiment of the invention said vehicle is suitable for use with any one of are the following cell types: keratinocyte, chondrocyte, osteoblast, endothelial cell, urothelial cell; epithelial cell.

In yet a further preferred embodiment of the invention said therapeutic vehicle comprises keratinocytes.

According to a third aspect of the invention there is provided a cosmetic vehicle, for use in tissue engineering, wherein said vehicle has integral therewith, or applied thereto, a cell culture surface obtained by plasma polymerisation or plasma co-polymerisation, to which at least one cell is reversibly attached, wherein the surface contains an acid functionality of at least 5% and the cell is a cell type selected from any one of the following cell types: keratinocytes, endothelial cells and epithelial cells.

According to a fourth aspect of the invention there is provided a method of preparing a surface according to any previous aspect or embodiment of the invention comprising:
i) mixing a selected ratio of acid containing monomer and a hydrocarbon in a gas feed;
ii) creating a plasma of said mixture; and
iii) coating a suitable substrate with said plasma to provide a surface polymer/copolymer retaining high acid functionality.

It will be apparent to one skilled in the art that the creation of a plasma can use either low or high power and either a continuous wave or pulsed plasma.

Preferably said plasma power is created using a plasma power of 0 -50W and a flow rate of 0 - 20 sccm, usually under continous wave conditions. However, in the instance of where a pulsed wave is used corresponding corrections are made to the plasma power and flow rate as is known by those skilled in the art.

In a preferred method of the invention said acid is acrylic acid and said hydrocarbon is a diene and especially a di-unsaturated alkene, for example 1,7-octadiene.

In a further preferred method of the invention said plasma comprises 50-100% unsaturated acid, for example, acrylic acid and 0-50% hexane or diene, (for example, 1,7-octadiene) in the gas feed.

In yet a further preferred embodiment of the invention said plasma comprises the following ratios of acid (eg acrylic acid) and hexane or diene(eg1,7-octadiene);

| Acid | | alkene |
|---|---|---|
| (eg Acrylic acid) | % | (eg 1,7-octadiene %) |
| 50 | | 50 |
| 60 | | 40 |
| 70 | | 30 |
| 80 | | 20 |
| 90 | | 10 |
| 100 | | 0 |

in the gas feed.

An embodiment of the invention will now described, by example only and with reference to the following tables and figures;
Table 1 shows the summary of XPS results PCPs formed from acrylic acid and 1,7 octadiene;
Table 2 shows the summary of XPS results from PPs prepared from propionic acid;
Table 3 shows the summary of XPS results for PPs prepared from pulsed acrylic acid;
Table 4 shows the summary of XPS results from PPs prepared from high power acrylic acid;
Table 5 shows the adherence of various surfaces to DED after 4 days in contact;
Figure 1 shows the attachment of keratinocytes to various surfaces;
Figure 2 shows the attachment of keratinocytes to high power acrylic acid, pulsed acrylic acid and propionic acid surfaces;
Figure 3 is a measure of keratinocyte retention on DED after transfer;
Figure 4(a) shows staining due to keratinocyte transfer to DED from Collagen I, carrier and hydrocarbon surfaces after 4 days in contact with DED;
Figure 4(b) shows staining due to keratinocyte transfer to DED from acid containing surfaces after 4 days in contact with DED;
Figure 5 shows staining due to keratinocyte transfer to DED from pulsed acrylic acid surfaces after 4 days in contact with DED;
Figure 6 shows staining due to keratinocyte transfer to DED from propionic acid surfaces after 4 days in contact with DED;
Figure 7 shows staining due to keratinocyte transfer from high power acrylic acid surfaces after 4 days in contact with DED;

### Materials and Methods

### Plasma Co-Polymerisation

Acrylic acid and 1,7-octadiene and propionic acid were obtained from Aldrich Chemical Co. (UK). All monomers were used as received, after several freeze-pump/thaw cycles. Polymerisation was carried out in a cylindrical reactor vessel (of 8 cm diameter and 50 cm in length), evacuated by a two stage rotary pump. The plasma was sustained by a radio-frequency (13.56 MHz) signal generator and amplifier inductively coupled to the reactor vessel. The base pressure in the reactor was 3×10⁻³ mbar.

Acrylic acid and 1,7-octadiene were co-polymerised at a plasma power of 2 W and a total flow rate of 2.0 sccm. Plasma co-polymers were deposited onto a carrier polymer, polyhydroxybutyrate (Goodfellow, Cambridge, UK) and clean aluminium foil (for XPS analysis). The pressure during co-polymerisation was typically 4.0×10⁻² mbar. A further polymerisation to deposit propionic acid was carried out using the same conditions. In addition, acrylic acid was deposited using pulsed plasma conditions. The plasma power was 50 W, using a duty cycle of 5 ms plasma on-time and 40 ms plasma off-time. The monomer flow rate was 2.0 sccm. Finally, acrylic acid was deposited under continuous wave high power conditions. The power used was 7.5 W with a flow rate of 2.0 sccm.

For all co-polymerisations, a deposition time of 20 minutes was used. The monomer mixtures were allowed to flow for a further 20 minutes after the plasma was switched off. This was done in an attempt to minimise the up-take of atmospheric oxygen by the deposits on exposure to the laboratory atmosphere.

### X-ray Photoelectron Spectroscopy

XP spectra were obtained on a VG CLAM 2 photoelectron spectrometer employing Mg K α X-rays. Survey scan spectra (0-1100 eV) and narrow spectra were acquired for each sample using analyser pass energies of 50 and 20 eV respectively. Spectra were acquired using Spectra 6.0 software (R. Unwin Software, Cheshire, UK). Subsequent processing was carried out with Scienta data processing software (Scienta Instruments, Uppsala, Sweden). The spectrometer was calibrated using the Au 4f 7/2 peak position at 84.00 eV, and the separation between the C 1s and F 1s peak positions in a sample of PTFE measured at 397.2 eV, which compares well with the value of 397.19 eV reported by Beamson and Briggs (13).

### Cell Culture

Normal human adult keratinocytes (obtained from breast reductions and abdominoplasties) were isolated from the dermal/epidermal junction as previously described (14). Cells were cultured in complete Green's media, which included cholera toxin (0.1 nM), hydrocortisone (0.4 µg/ml), EGF (10 ng/ml), adenine (1.8×10⁻⁴ M), triiodo-1-thyronine (2×10⁻⁷ M), insulin (5mg/ml), transferrin (5 µg/ml), glutamine (2×10⁻³ M), fungizone (0.625 µg/ml), penicillin (1000 IU/ml), streptomycin (1000 µg/ml) and 10 per cent fetal calf serum. Cells were cultured at 37°C, in a 5% CO₂ atmosphere. Total cell counts and viable cell number were determined using Trypan Blue Stain and a standard hemocytometer chamber.

Only freshly isolated cells were used for the cell culture experiments. Collagen coated carrier polymer samples were prepared by air drying a solution of collagen I (32µg/cm²) in 0.1 M acetic acid (200µg/ml) in a laminar flow cabinet overnight.

Cells were seeded at densities of 12.0×10⁶ cells/ml onto triplicates of the surfaces using a 10 mm diameter stainless steel ring to keep the samples flat in 6 well tissue culture plates. After 24h in culture, the keratinocyte attachment on one sample from each triplicate was determined using an MTT-ESTA assay(15). This estimates the viable cell number, the assay having previously shown to parallel increases in cell number for human keratinocytes (16). Cells were incubated with 0.5 mg ml⁻¹ of MTT in PBS for 40 min. The stain was then eluted with acidified isopropyl alcohol. An optical density measurement was then made at 540 nm with a protein reference wavelength of 630 nm which was subtracted.

The remaining two samples from each triplicate were placed in contact with DED and Green's media added so that the surfaces sat at the air/liquid interface. The DED/surface wound bed models were placed in an incubator at 37°C for 4 days, after which the surfaces were separated from the DED and the level of cell transfer from surface to DED assessed using the MTT assay, as described above. MTT of the DED required that the DED was incubated with MTT for 120 mins before elution of the stain.

### Results

### XPS characterisation

XP survey scan spectra of PCPs prepared from acrylic acid and 1,7-octadiene revealed only carbon and oxygen in the deposits. The O/C ratios are shown in Table 1. The O/C ratio increased as the mole fraction of acrylic acid in the monomer feed increased. The C 1s core level spectrum of the PCP was peak fitted for various oxygen-containing functionalities. First, spectra were corrected for sample charging, setting the hydrocarbon signal to 285 eV. The following functionalities were then fitted: alcohol/ether (C-OH/R) at a shift of +1.5 eV; carbonyl (C=O) at +3.0 eV; carboxylic acid/ester (COOH/R) at +4.0 eV; and a β-shifted carbon bonded to carboxylate (C-COOH/R) at +0.7 eV. The results of peak fitting are shown in Table 1 and an example peak fit (Fₐₐ/Fₜₒₜ = 1). In the peak fit the FWHM of component peaks were kept equal and were in the range 1.4-1.6 eV. The Gaussian to Lorentzian ratio (G/L) of the component peaks were also kept constant and were in the range 0.8-0.9. While XPS cannot distinguish between carboxylic acid and ester groups, grazing angle infra-red spectroscopy of plasma polymerised acrylic acid has shown, that at the low powers employed in this study, the carboxylate peak in the XP spectra can be assigned to carboxylic acid rather than ester (10). Other carbon-oxygen functionalities present in the PCPs (besides carboxylic acid) include carbonyl and alcohol/ether. These arise as a result of fragmentation of the monomer in the plasma. Reaction between the deposit and water desorbed from the walls of the plasma vessel (during polymerisation) and atmospheric oxygen and water (after polymerisation) also contribute. The C-OH/R peak is thought to be predominantly hydroxyl. In a previous study we examined the identity of the oxygen-containing functionalities in PCPs of acrylic acid/1,7-octadiene (prepared with varying molar fractions of acrylic acid in the monomer feed) in more detail (11) Based on this study, we believe that on the PCP surface, keratinocytes respond to the carboxylic acid functionality, and not C-OH. The latter has to be present in high concentrations (25%) to promote cell attachment (8).

XP spectra from i) continuous wave depositions of propionic acid, ii) pulsed acrylic acid, and iii) high power acrylic acid, also revealed only carbon and oxygen in the deposits, and were fitted using the same criteria outlined above for acrylic acid. The results of the curve fitting for propionic acid, pulsed acrylic acid and high power acrylic acid are shown in Tables 2, 3 and 4 respectively. Based on previous studies of pulsed plasmas in our laboratory it is expected that lower duty cycle in pulsed plasmas would yield even higher values of carboxyl retention (21). It is clear by comparing Tables 1 and 4 that increasing the plasma power leads to a drop in retention of carboxyl functionality of ∼50%, and a corresponding increase in alcohol/ether and carbonyl functionalities.

**Table 1 Summary of XPS results for PCPs prepared from acrylic acid and 1,7 octadiene**

| | | % functionality in C1 s core level | | | |
|---|---|---|---|---|---|
| Fₐₐ/Fₜₒₜ | O/C ratio | C-C, C-H | C-OR | C=O | COOH/R |
| 0 | 0.04 | 95.8 | 4.7 | - | - |
| 0.25 | 0.11 | 88.4 | 5.7 | 1.0 | 2.6 |
| 0.5 | 0.16 | 87.1 | 1.4 | 1.2 | 5.4 |
| 1.0 | 0.51 | 50.4 | 6.4 | 1.0 | 21.1 |
| carrier | 0.47 | 52.2 | 1.2 | 16.0 | 16.0 |

A β-shifted carbon bonded to carboxylate ((C-COOH/R) at +0.7 eV from the hydrocarbon) of equal magnitude to the carboxylate has been added to the peak fit.

**Table 2 Summary of XPS results for PPs prepared from propionic acid**

| | | % functionality in C 1s core level | | | |
|---|---|---|---|---|---|
| Fₚₐ/Fₜₒₜ | O/C ratio | C-C, C-H | C-OR | C=O | COOH/R |
| 1.0 | 0.58 | 52.0 | 8.5 | 3.8 | 18.0 |

A β-shifted carbon bonded to carboxylate ((C-COOH/R) at +0.7 eV from the hydrocarbon) of equal magnitude to the carboxylate has been added to the peak fit.

**Table 3 Summary of XPS results for pulsed PPs of acrylic acid**

| | | % functionality in C 1s core level | | | |
|---|---|---|---|---|---|
| Fₐₐ/Fₜₒₜ | O/C ratio | C-C, C-H | C-OR | C=O | COOH/R |
| 1.0 | 0.51 | 53.9 | 8.3 | 1.2 | 18.3 |

A β-shifted carbon bonded to carboxylate ((C-COOH/R) at +0.7 eV from the hydrocarbon) of equal magnitude to the carboxylate has been added to the peak fit.

**Table 4 Summary of XPS results for PPs of high power acrylic acid**

| | | % functionality in C 1s core level | | | |
|---|---|---|---|---|---|
| Fₐₐ/Fₜₒₜ | O/C ratio | C-C, C-H | C-OR | C=O | COOH/R |
| 1.0 | 0.47 | 54.2 | 14.5 | 8.6 | 11.4 |

A β-shifted carbon bonded to carboxylate ((C-COOH/R) at +0.7 eV from the hydrocarbon) of equal magnitude to the carboxylate has been added to the peak fit.

### Cell attachment on surfaces

For all surfaces, after isolation of the keratinocytes a cell count was performed using a hemocytometer which showed 97% cell viability (2.5 x 10⁷ total cells).
After 24 h the surfaces were examined using an MTT assay.

### i) Acrylic acid/1,7-octadiene:

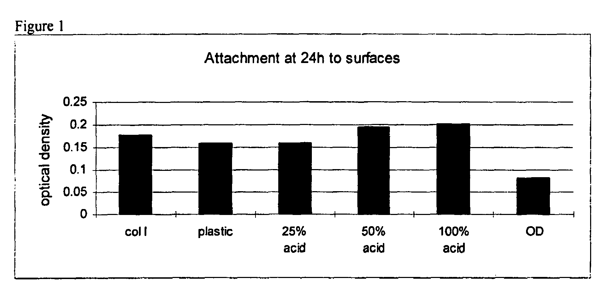

The results are shown in Fig. 1. The data show that acid containing surfaces prepared with 50% and 100% acrylic acid in the monomer flow performed slightly better than Collagen I. The surface made with 25% acid in the flow was comparable to TCPS, whilst keratinocyte attachment on the hydrocarbon surface was poor.

### ii) Propionic Acid, High Power Acrylic Acid, Pulsed Acrylic Acid.

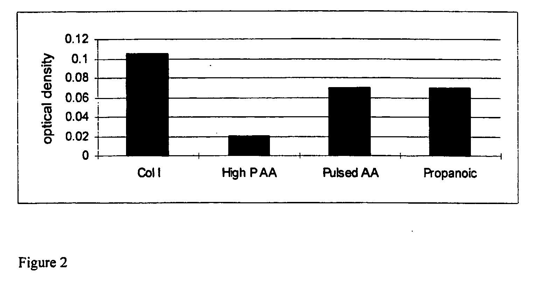

The results are shown in Figure 2. There was cell attachment to all surfaces, although Collagen I showed the highest level of attachment. Although the levels of cell attachment are not a predictor for the degree of subsequent transfer from the surfaces to DED, it is important to note that the surfaces produced using different precursor monomers and/or plasma conditions do support the attachment of keratinocytes. Cell attachment is clearly a pre-requisite for subsequent transfer to be successful.

### Transfer of cells to DED

Table 5 summarises the results of separating the carrier polymer surfaces from the DED. The collagen I surface and the surface prepared with 100% in the gas flow were well adhered to the DED, indicating that substantial transfer of keratinocytes from the surface to the DED had occurred. Surfaces with lower amounts of acid in the monomer flow were less well adhered, whilst the carrier and hydrocarbon surfaces readily peeled apart from the DED, indicating lesser degrees of cell transfer had taken place.

**Table 5 Adherence of surfaces to DED after 4 days in contact**

| | |
|---|---|
| Collagen I | Well adhered |
| Hydrocarbon | Peeled apart easily-no adherence |
| Carrier | Peeled apart easily-no adherence |
| 25% Acrylic Acid | Adhered |
| 50% Acrylic Acid | Adhered, but less than 25% surface |
| 100% Acrylic Acid | Well adhered |

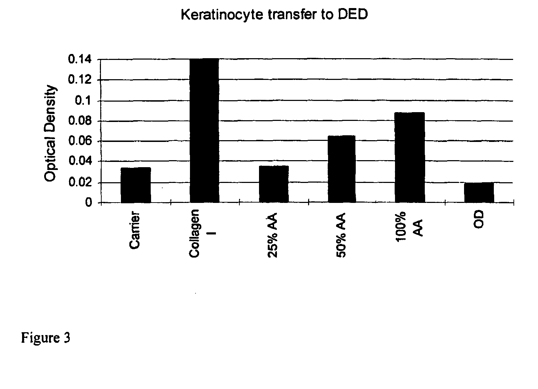

After 4 days of juxtaposition of the surfaces and the DED, the two were separated and Figure 2 shows the results of the MTT assay on the surface and Figure 3 of the MTT assay on the DED. The optical density of the cells remaining on the surfaces was extremely low in all cases compared to that seen for cells transferred to the DED. Cells grown on collagen 1 exhibited the highest value when examined for transfer to the DED, approximately 4 times greater than that seen for with carrier alone. For cells grown on the 25% acid treated surface transfer was comparable to that seen with cells grown on carrier alone. Cells grown on the 50% and 100% acid treated, surfaces showed however significantly greater transfer to the DED. Cells grown on the hydrocarbon treated surface showed very little transfer to the DED.

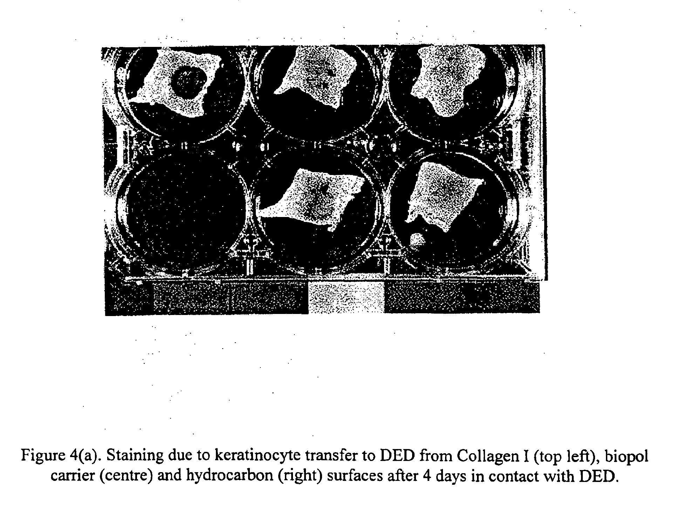

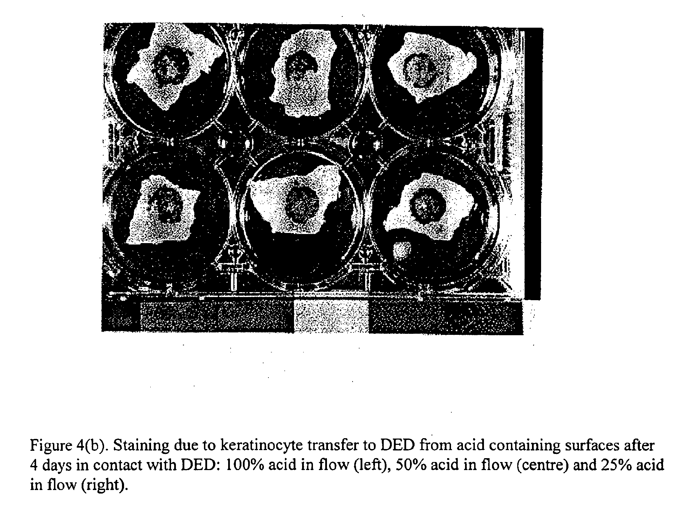

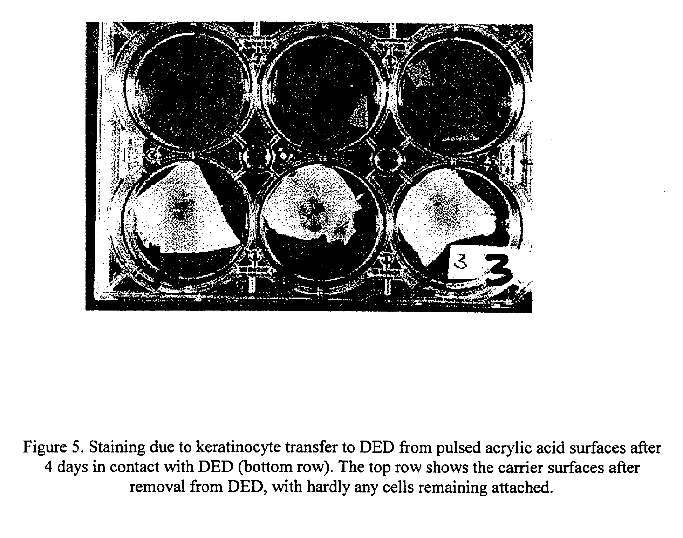

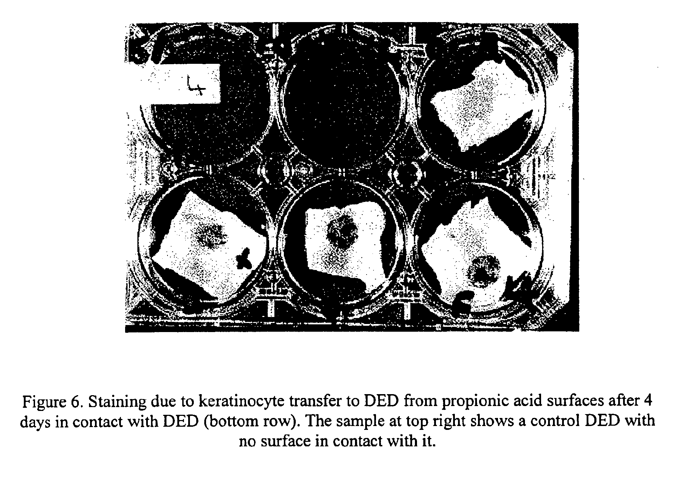

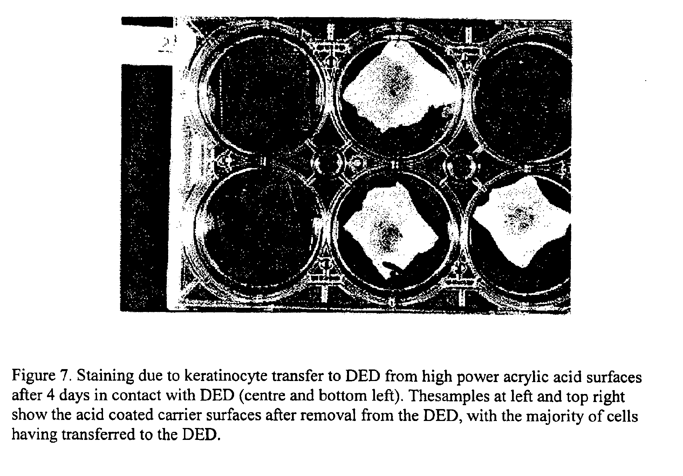

Photographic evidence of keratinocyte transfer from acrylic acid/1,7-octadiene PPs to DED is shown in Figure 4a and Figure 4b. The hydrocarbon (1,7-octadiene) and carrier (biopol) surfaces are unstained, whilst the Collagen I and acid containing surfaces exhibit the characteristic purple staining due to keratinocytes on the DED. Figure 5 shows the DED staining due to cell transfer from a pulsed acrylic acid PP. Figure 6 shows the same results using a propionic acid PP. Shown in Figure 7 is the DED staining due to transfer from a high power acrylic acid PP (deposited on a nonwoven fabric).

### Discussion

The purpose of this study was to extend previous work from this laboratory, which had disclosed the use of PCP surfaces for keratinocyte attachment and proliferation, but not addressed the transfer of these cells to DED. Keratinocytes represent a particular challenge for such studies because they will undergo irreversible terminal differentiation on many substrates. Such cells lose the capacity to migrate or form colonies - properties which are required in considering transfer of keratinocytes from supporting surfaces to wound beds to achieve re-epithelialization.

Accordingly, our aim was to examine to what extent surfaces which promoted attachment would encourage transfer of cells in a simple in vitro wound model.

Human keratinocytes were successfully cultured on PCP surfaces containing varying concentrations of carboxylic acid groups, with the number of cells attached being comparable to the performance of cells on collagen I, a preferred substrate for keratinocyte culture.

The use of a hydrocarbon plasma polymer as a negative controls is important because a previous study has raised doubts about the suitability of TCPS as a control (17). These concerns have arisen because of the surface treatments given to TCPS during manufacture, which may render TCPS unstable to aqueous solutions depending on the level of oxidation at the surface. It is unclear whether different batches of TCPS receive precisely the same amount of surface oxidation, or if this surface oxidation is susceptible to ageing.

Although the dependence of cell attachment on functional group concentration is yet to be fully explored, keratinocytes have been previously shown to have enhanced attachment on surfaces with low 2-3% amounts amounts of acid functionality (11). However, in this study attachment is also shown to be high on a surface containing 21 % acid. It should be recalled that the acid PCPs also contain other O-C functional groups, predominantly C-OH. Even so, our previous studies have demonstrated that acid PCPs are comparable to collagen I in terms of degree of confluency and cell number (as determined by DNA assay).

In aqueous media, the acid PCPs can hydrate, as we will demonstrate elsewhere (18). The stability of acid PCPs has been shown to be dependent on the concentration of acrylic acid in the monomer flow. High concentrations of acid (>60% of the total flow) result in less stable surfaces. This requirement led to the development of low concentration acid surfaces (<5%) as being labelled "ideal" for promoting attachment and subsequent proliferation. However, with regard to cell transfer from acid surfaces, different criteria are likely to apply. Whilst low concentrations of acid groups impart stability to the surface, the keratinocytes may be sufficiently well attached that transfer is inhibited. This assertion is borne out in the results of the transfer experiments, where 25% acid flow in the monomer feed (2.6% carboxylic acid at the PCP surface) showed the lowest degree of transfer to DED. In contrast, with 100% acid flow in the monomer (>20% carboxylic acid at the PCP surface), transfer of cells was significantly higher. These surfaces were only outperformed by Collagen I. With 50% acid in the monomer flow, transfer was intermediate between the high and low acid functionality surfaces, as would be expected. These results indicate that the optimum surfaces for promoting attachment and proliferation may not be those which result in the largest degree of keratinocyte transfer from PCPs to DED. The low amount of transfer from the hydrocarbon PP confirm that such a surface is not capable of supporting keratinocytes in a proliferative state. Although the dependence of cell transfer on functional group concentration is yet to be fully explored, keratinocytes show enhanced transfer from surfaces with high amounts of acid functionality. It is clear therefore that there exists a compromise between surfaces which promote proliferation (low acid functionality), and those which promote transfer (high acid functionality).

In serum-containing medium, it has been shown that cells respond to an adsorbed layer of protein, rather than directly to the substratum itself (19). This interfacial protein layer adsorbs (almost) spontaneously. The differences in cell response to the substrata under investigation suggest that there are either changes in the composition of the protein films that adsorb or in the activities of these proteins after adsorption, or a combination of both of these. Cell attachment has been shown to be supported by a number of adhesive proteins, such as fibronectin and vitronectin. Tidwell et al (12) have shown differences in the protein layers that develop on SAMs with alkanethiolates of different terminal chemistries and that these in turn support different levels of bovine aortic endothelial cell attachment. Whilst cell attachment and spreading are important conditions for cell proliferation, they are not exclusive conditions. Serum is also a source of growth factors and these have been shown to be essential for the proliferation of primary mammalian cells. It has been suggested that the adsorption of growth factors onto extracellular matrix material plays a role in their activation (20).

The results show that the carboxylic acid functionality can be provided from a wide range of starting monomers. Acrylic acid is a member of the unsaturated family of organic acids, whilst propionic acid is a saturated organic acid. It is therefore expected that any organic acid could be used to manufacture PP surfaces capable of exhibiting keratinocyte attachment and subsequent transfer to DED, provided that the monomer is sufficiently volatile to flow through the plasma chamber. Furthermore, the results indicate that a wide range of plasma conditions are capable of preparing surfaces that promote the required cell attachment and transfer. We have shown that under continuous wave conditions both low and high power regimes can be used to successfully to prepare PPs with the desired properties. In addition, pulsing the plasma provides another route to deposit acid functionalities on a surface. Another consideration is that a range of carrier surfaces can be used for plasma deposition. In this study a biodegradable carrier (biopol), and a non-biodegradable carrier (polypropylene, Delnet - supplied by AET Specialty Nets) have both shown that they can be coated with a PP that promotes keratinocyte cell transfer to DED. The data suggest that whilst the carrier films may differ in their properties (e.g. solubility, absorbency), it is primarily the PP deposit on these carriers that influences cell behaviour (although topographical effects such as weave / pore size may mean that the PP coating is not a uniform 'flat' surface, depositing as it will on the contours of the carrier. Cells may therefore attach in places where they may not come into contact with DED when the PP coated carrier is applied to it). It is envisaged that any surgical use of the transfer phenomenon would require a film-like carrier rather than a mesh, to ensure maximum attachment of cells at the PP surface, rather than within the coated contours of the carrier material.

Based upon the above discussion, it is evident that the success of the acid PCPs in supporting keratinocyte attachment and transfer is multi-factorial. However, our results would indicate that keratinocyte attachment and transfer are promoted specifically by the carboxylic acid functionality. This is most probably through control of the interfacial protein layer that forms from serum.

PCP surfaces containing high concentrations of acid groups (typically carboxylic) encouraged keratinocyte attachment and transfer to DED compared to hydrocarbon surfaces. The initial attachment of cells on surfaces containing ∼20% acid groups was comparable to that of cells on collagen I substrates after 24h in culture.

Cell transfer from PCPs to DED was greatest for surfaces containing high concentrations of carboxylic acid functionality, although transfer was also observed from surfaces with low acid functionality concentration. Cell transfer was achieved using PPs deposited from both saturated and unsaturated organic acids. Under continuous wave conditions, both low and high power regimes were capable of producing a PP that promoted cell transfer. Pulsed plasmas also provided a route to manufacturing transfer-promoting PP surfaces.

### References

**1.** H.O. Rennekampff, V. Kiessig and J.F. Hansbrough, "Current concepts in the development of cultured skin replacements," J. Surgical Res., 62(2), 288-295 (1996).
**2.** M.D. Harriger, G.D. Warren, D.G. Greenhalgh, R.J. Kagan and S.T. Boyce, "Pigmentation and microanatomy of skin regenerated from composite grafts of cultured cells and biopolymers applied to full thickness bum wounds," Transplantation, 59(5), 702-707 (1995).
**3.** K. Smetana, J. Vacik and B. Dvorankova, "Interactions of cells with polymers," Macromolecular Symposia, 109, 145-153 (1996).
**4.** B. Dvorankova, K. Smetana, R. Konigova, H. Singerova, J. Vacik, M. Jelinkova, Z. Kapounkova and M. Zahradnik, "Cultivation and grafting of human keratinocytes on a poly(hydroxyethyl methacrylate) support to the wound bed-a clinical study," Biomaterials, 19(1-3), 141-146 (1998).
**5.** D.B. Haddow, R.M. France, R.D. Short, S. MacNeil, R.A. Dawson, G.J. Leggett and E. Cooper, "Comparison of proliferation and growth of human keratinocytes on plasma copolymers of acrylic acid/1,7-octadiene and self assembled monolayers," J. Biomed. Mater. Res., 47, 379-387 (1999).
**6.** R. A. Dawson, N. J.Goberdhan, E. Freedlander and S. MacNeil, "Influence of extracellular-matrix proteins on human keratinocyte attachment, proliferation and transfer to a dermal wound model," Burns, 22, 93-100 (1996).
**7.** E. Cooper, R. Wiggs, D. A. Hutt, L. Parker, G. J. Legget and T. L. Parker, "Rates of attachment of fibroblasts to self-assembled monolayers formed by adsorption of alkylthiols onto gold surfaces," J. Mater. Chem., 7(3), 435-441 (1997). **7b**. C. D. Bain, E. B. Troughton, Y-T. Tao, J. Evall, G. M. Whitesides and R. G. Nuzzo, J.. Am. Chem. Soc., 111, 321, (1989)
**8.** R.M. France, R.D. Short, E. Duval, R.A. Dawson and S. MacNeil, "Plasma copolymerisation of allyl alcohol/1,7-octadiene: surface characterisation and attachment of human keratinocytes," Chem. of Mater., 10(4), 1176-1183 (1998).
**9.** C. A. Scotchford, E. Cooper, G. J. Leggett and S. Downes, "Growth of Human Osteoblast-Like Cells on Alkanethiol-on-gold Self-assembled Monolayers: the Effect of Surface Chemistry", J. Biomed. Mater. Res., 41, 431-442 (1998).
**10.** L. O'Toole, A. J. Beck and R.D. Short, "Characterisation of plasma-polymers of acrylic acid and propanoic acid," Macromolecules, 29(15), 5172-5177 (1996).
**11.** R.M. France, R.D. Short, R.A. Dawson and S.MacNeil, "Attachment of human keratinocytes to plasma co-polymers of acrylic acid/1,7-octadiene and allyl amine/1,7-octadiene," J. Mater. Chem, 8(1), 37-42 (1998).
**12.** C.D. Tidwell, S.I. Ertel, B.D. Ratner, B.J. Tarasevich, S. Artre and D.L. Allara, "Endothelial cell growth and protein adsorption on terminally functionalised self-assembled monolayers of alkanethiolates on gold," Langmuir, 13(13), 3404-3413 (1997).
**13.** G. Beamson and D. Briggs, eds., High Resolution XPS of Organic Polymers: The Scienta ESCA300 Handbook, John Wiley and Sons, Chichester, 1992.
**14.** N. J. Goberdhan, M. Edgecombe, E. Freedlander and S. MacNeil, "Extracellular-matrix proteins induce changes in intracellular calcium and cyclic AMP signalling systems in cultured human keratinocytes," Burns, 23(2), 122-130 (1997).
**15.** P.A. Ealey, M.E. Yateman, S.J. Holt and N.J. Marshall, J. Mol. Enocrinol., 1,1 (1988).
**16.** S. MacNeil, R.A. Dawson and G. Crocker, Br. J. Dermatol., 128, 143 (1993).
**17.** R. M. France and R. D. Short, "Plasma treatment of polymers-effects of energy transfer from an argon plasma on the surface chemistry of poly(styrene), low density poly(ethylene), poly(propylene) and poly(ethylene terephthalate)," J. Chem. Soc., Faraday Trans., 93(17), 3173-3178 (1998).
**18.** R. M. France, C. J. Roberts, S. J. Tendler, P. M. Williams, R. D. Short R Daw, "Plasma Copolymers of Acrylic Acid /1, 7-Octadiene: An investigation of hydration and protein adsorption using surface plasmon reseonance" in preparation.
**19.** H. J. Griesser, R. C. Chatelier, T. R. Gengenbach, G. Johnson and J. G. Steele, "Growth of human cells on plasma-polymers-putative role of amine and amide groups," J. Biomat. Sci., Polym. Ed., 5(6), 531-554 (1994).
**20.** T. N. Wight, M. G. Kinsella and E. E. Quarnstorm, "The role of proteoglycans in cell adhesion migration and proliferation" Ann. Rev. Cell Biol. 8, 365-393 (1992).
21. D. B. Haddow, A. J. Beck, J. D. Whittle, R. M. France and R. D. Short, "A mass spectral investigation of the plasma-phase of a pulsed plasma of acrylic acid", submitted to J. Vac. Sci., April 2000.

## Claims

1. A therapeutic vehicle, wherein said vehicle has integral therewith, or applied thereto, a cell culture surface obtained by plasma polymerisation or plasma co-polymerisation, to which at least one cell is reversibly attached, wherein the surface contains an acid functionality of at least 5% and the cell is a cell type selected from any one of the following cell types: keratinocytes, endothelial cells and epithelial cells, for use as a medicament.

2. A therapeutic vehicle, for use in wound healing, wherein said vehicle has integral therewith, or applied thereto, a cell culture surface obtained by plasma polymerisation or plasma co-polylnerisation, to which at least one cell is reversibly attached, wherein the surface contains an acid functionality of at least 5% and the cell is a cell type selected from any one of the following cell types: keratinocytes, endothelial cells and epithelial cells.

3. A cosmetic vehicle, for use in tissue engineering, wherein said vehicle has integral therewith, or applied thereto, a cell culture surface obtained by plasma polymerisation or plasma co-polymerisation, to which at least one cell is reversible attached, wherein the surface contains an acid functionality of at least 5% and the cell is a cell type selected from any one of the following cell types: keratinocytes, endothelial cells and epithelial cells.

4. A vehicle according to any preceding claim, wherein said vehicle is selected from the group consisting of: a prosthesis; implant; matrix; stent; gauze; bandage; plaster; biodegradable matrix; and polymeric film.

5. A vehicle according to claim 4, wherein the vehicle is a polymeric film.

6. A vehicle according to any preceding claim, wherein said surface acid functionality is between 5% and 20%.

7. A vehicle according to any one of claims 1-5, wherein said surface acid functionality is greater than 20%.

8. A vehicle according to any preceding claim, wherein said surface acid functionality is provided by one of the following: carboxylic acid, propionic acid and acrylic acid.

9. A vehicle according to any one of claims 1-7, wherein said surface is provided by coating a substrate with a plasma polymer or co-polymer of an acid containing monomer.

10. A vehicle according to claim 9, wherein said co-polymer is a mixture of acrylic acid and a hydrocarbon.

11. A vehicle according to claim 10, wherein said hydrocarbon is 1,7-octadiene.

12. A vehicle according to either claim 10 or 11, wherein acrylic acid is provided at 50-100%, and 1,7-octadiene is provided at 0-50% in a gas feed.

13. A vehicle according to any preceding claim, wherein the at least one cell is of mammalian origin, and wherein said surface is suitable for use with cells of mammalian origin.

14. A vehicle according to claim 13, wherein said mammalian cells are human.

15. A vehicle according to either claim 13 or 14, wherein said surface is suitable for use with any one of the following cell types: keratinocytes; endothelial cells; and epithelial cells.

16. A vehicle according to claim 15, wherein the cell type is a keratinocyte.

17. A vehicle according to claim 2, for use in treating a chronic wound.

18. A vehicle according to claim 2, for use in treating an acute wound.

19. A vehicle according to claim 2, for use in treating cutaneous wounds.

20. A vehicle according to claim 19, for use in treating diabetic or venous ulcers.

## Patentansprüche

1. Therapeutischer Träger, wobei der Träger eine damit integrale oder darauf aufgebrachte Zellkulturoberfläche aufweist, die mittels Plasmapolymerisation oder Plasmacopolymerisation erhalten wurde und an die mindestens eine Zelle reversibel angelagert ist, wobei die Oberfläche eine Säurefunktionalität von mindestens 5 % enthält und die Zelle ein Zelltyp ist, der aus einem beliebigen der folgenden Zelltypen ausgewählt ist: Keratinozyten, Endothelzellen und Epithelzellen, zur Verwendung als ein Arzneimittel.

2. Therapeutischer Träger zur Verwendung in der Wundheilung, wobei der Träger eine damit integrale oder darauf aufgebrachte Zellkulturoberfläche aufweist, die mittels Plasmapolymerisation oder Plasmacopolymerisation erhalten wurde und an die mindestens eine Zelle reversibel angelagert ist, wobei die Oberfläche eine Säurefunktionalität von mindestens 5 % enthält und die Zelle ein Zelltyp ist, der aus einem beliebigen der folgenden Zelltypen ausgewählt ist: Keratinozyten, Endothelzellen und Epithelzellen.

3. Kosmetischer Träger zur Verwendung in der Gewebezüchtung, wobei der Träger eine damit integrale oder darauf aufgebrachte Zellkulturoberfläche aufweist, die mittels Plasmapolymerisation oder Plasmacopolymerisation erhalten wurde und an die mindestens eine Zelle reversibel angelagert ist, wobei die Oberfläche eine Säurefunktionalität von mindestens 5 % enthält und die Zelle ein Zelltyp ist, der aus einem beliebigen der folgenden Zelltypen ausgewählt ist: Keratinozyten, Endothelzellen und Epithelzellen.

4. Träger nach einem der vorhergehenden Ansprüche, wobei der Träger aus der Gruppe bestehend aus einer Prothese; einem Implantat; einer Matrix; einem Stent; einer Gaze; einem Verband; einem Pflaster; einer biologisch abbaubaren Matrix und einer Polymerfolie ausgewählt ist.

5. Träger nach Anspruch 4, wobei es sich bei dem Träger um eine Polymerfolie handelt.

6. Träger nach einem der vorhergehenden Ansprüche, wobei die Oberflächen-Säurefunktionalität zwischen 5 % und 20 % liegt.

7. Träger nach einem der Ansprüche 1 - 5, wobei die Oberflächen-Säurefunktionalität höher als 20 % ist.

8. Träger nach einem der vorhergehenden Ansprüche, wobei die Oberflächen-Säurefunktionalität von einer der folgenden Säuren bereitgestellt wird: Carbonsäure, Propionsäure und Acrylsäure.

9. Träger nach einem der Ansprüche 1 - 7, wobei die Oberfläche bereitgestellt wird, indem ein Substrat mit einem Plasmapolymer oder -copolymer eines säurehaltigen Monomers beschichtet wird.

10. Träger nach Anspruch 9, wobei es sich bei dem Copolymer um ein Gemisch von Acrylsäure und einem Kohlenwasserstoff handelt.

11. Träger nach Anspruch 10, wobei es sich bei dem Kohlenwasserstoff um 1,7-Octadien handelt.

12. Träger nach Anspruch 10 oder 11, wobei Acrylsäure zu 50 - 100 % und 1,7-Octadien zu 0 - 50 % in einer Gaszufuhr bereitgestellt werden.

13. Träger nach einem der vorhergehenden Ansprüche, wobei die mindestens eine Zelle von einem Säugetier stammt und wobei die Oberfläche zur Verwendung mit Zellen von einem Säugetier geeignet ist.

14. Träger nach Anspruch 13, wobei die Säugetierzellen von einem Menschen stammen.

15. Träger nach Anspruch 13 oder 14, wobei die Oberfläche zur Verwendung mit einem beliebigen der folgenden Zelltypen geeignet ist: Keratinozyten; Endothelzellen und Epithelzellen.

16. Träger nach Anspruch 15, wobei es sich bei dem Zelltyp um einen Keratinozyten handelt.

17. Träger nach Anspruch 2 zur Verwendung in der Behandlung einer chronischen Wunde.

18. Träger nach Anspruch 2 zur Verwendung in der Behandlung einer akuten Wunde.

19. Träger nach Anspruch 2 zur Verwendung in der Behandlung einer Hautwunde.

20. Träger nach Anspruch 19 zur Verwendung in der Behandlung diabetischer oder venöser Ulzera.

## Revendications

1. Véhicule thérapeutique, ledit véhicule ayant, intégré à celui-ci, ou appliqué sur celui-ci, une surface de culture cellulaire obtenue par polymérisation plasma ou copolymérisation plasma, sur laquelle au moins une cellule est fixée de manière réversible, la surface contenant une fonctionnalité acide d'au moins 5 % et la cellule étant un type cellulaire choisi parmi l'un quelconque des types cellulaires suivantes : kératinocytes, cellules endothéliales et cellules épithéliales, destiné à être utilisé en tant que médicament.

2. Véhicule thérapeutique, destiné à être utilisé pour la cicatrisation des plaies, ledit véhicule ayant, intégré à celui-ci, ou appliqué sur celui-ci, une surface de culture cellulaire obtenue par polymérisation plasma ou copolymérisation plasma, sur laquelle au moins une cellule est fixée de manière réversible, la surface contenant une fonctionnalité acide d'au moins 5 % et la cellule étant un type cellulaire choisi parmi l'un quelconque des types cellulaires suivants: kératinocytes, cellules endothéliales et cellules épithéliales.

3. Véhicule cosmétique, destiné à être utilisé pour l'ingénierie tissulaire, ledit véhicule ayant, intégré à celui-ci, ou appliqué sur celui-ci, une surface de culture cellulaire obtenue par polymérisation plasma ou copolymérisation plasma, sur laquelle au moins une cellule est fixée de manière réversible, la surface contenant une fonctionnalité acide d'au moins 5 % et la cellule étant un type cellulaire choisi parmi l'un quelconque des types cellulaires suivants: kératinocytes, cellules endothéliales et cellules épithéliales.

4. Véhicule selon l'une quelconque des revendications précédentes, ledit véhicule étant choisi dans le groupe constitué par : une prothèse ; un implant ; une matrice ; une endoprothèse ; une gaze hydrophile ; un bandage ; un emplâtre ; une matrice biodégradable ; et un film polymère.

5. Véhicule selon la revendication 4, dans lequel le véhicule est un film polymère.

6. Véhicule selon l'une quelconque des revendications précédentes, dans lequel ladite fonctionnalité acide de surface est comprise entre 5 % et 20 %.

7. Véhicule selon l'une quelconque des revendications 1 à 5, dans lequel ladite fonctionnalité acide de surface est supérieur à 20 %.

8. Véhicule selon l'une quelconque des revendications précédentes, dans lequel ladite fonctionnalité acide de surface est assurée par l'un des suivants : acide carboxylique, acide propionique et acide acrylique.

9. Véhicule selon l'une quelconque des revendications 1 à 7, dans lequel ladite surface est obtenue par le revêtement d'un substrat avec un polymère plasma ou un copolymère plasma d'un monomère contenant un acide.

10. Véhicule selon la revendication 9, dans lequel ledit copolymère est un mélange d'un acide acrylique et d'un hydrocarbure.

11. Véhicule selon la revendication 10, dans lequel ledit hydrocarbure est le 1,7-octadiène.

12. Véhicule selon la revendication 10 ou 11, dans lequel l'acide acrylique est introduit à raison de 50 à 100 %, et le 1,7-octadiène est introduit à raison de 0 à 50 % dans une alimentation en gaz.

13. Véhicule selon l'une quelconque des revendications précédentes, dans lequel la ou les cellules sont d'origine mammalienne, et dans lequel ladite surface est appropriée pour être utilisée avec des cellules d'origine mammalienne.

14. Véhicule selon la revendication 13, dans quel lesdites cellules de mammifère sont humaines.

15. Véhicule selon la revendication 13 ou 14, dans lequel ladite surface est appropriée pour être utilisée avec l'un quelconque des types cellulaires suivants : kératinocytes ; cellules endothéliales ; et cellules épithéliales.

16. Véhicule selon la revendication 15, dans lequel le type cellulaire est un kératinocyte.

17. Véhicule selon la revendication 2, destiné à être utilisé pour le traitement d'une plaie chronique.

18. Véhicule selon la revendication 2, destiné à être utilisé pour le traitement d'une plaie aiguë.

19. Véhicule selon la revendication 2, destiné à être utilisé pour le traitement de plaies cutanées.

20. Véhicule selon la revendication 19, destiné à être utilisé pour le traitement d'ulcères diabétiques ou d'ulcères veineux.
